# EUROPEAN PATENT APPLICATION

(11) **EP 3 711 802 A1**
(43) Date of publication of application: **23.09.2020**
(21) Application number: 19164438.4
(22) Date of filing: 21.03.2019
(51) Int. Cl.: A61M 15/06, A61M 11/06, A61M 16/10, A61M 11/04, A24F 47/00, A61M 11/02

(54) **AEROSOL DELIVERY SYSTEM**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

An aerosol delivery system has an air duct passing through it, from an inlet to a mouthpiece. The air duct passes through a mesh which receives aerosol precursor from a reservoir, so that, as the air passes through the mesh, the aerosol precursor on the mesh is atomised. The atomised aerosol precursor sprays from the mesh onto a heater which heats and vaporises the atomised aerosol precursor to form a mixture of vapour and air in the air duct at the heater. The mixture of vapour and air may then be drawn to the mouthpiece through the air duct. Thus, the user may receive a mixture of air and vapour by drawing air through the air duct. The heater receives a spray of atomised aerosol precursor, thereby making vaporisation of the aerosol precursor straightforward. There may be a wick between the reservoir and the mesh for regulating the transfer of aerosol precursor from the reservoir to the mesh.

## Description

### Field of the Invention

The present invention relates to an aerosol delivery system, and an aerosol-generation apparatus for an aerosol delivery system. The present invention preferably relates to an aerosol delivery system including a heater configured to heat an aerosol precursor to generate an aerosolised composition for inhalation by a user, and to an aerosol-generation apparatus therefor.

### Background

Pharmaceutical medicament, physiologically active substances and flavourings for example may be delivered to the human body by inhalation through the mouth and/or nose. Such material or substances may be delivered directly to the mucosa or mucous membrane lining the nasal and oral passages and/or the pulmonary system. For example, nicotine is consumed for therapeutic or recreational purposes and may be delivered to the body in a number of ways. Nicotine replacement therapies are aimed at people who wish to stop smoking and overcome their dependence on nicotine. Nicotine is delivered to the body in the form of aerosol delivery devices and systems, also known as smoking-substitute devices or nicotine delivery devices. Such devices may be non-powered or powered.

Devices or systems that are non-powered may comprise nicotine replacement therapy devices such as "inhalators", e.g. Nicorette® Inhalator. These generally have the appearance of a plastic cigarette and are used by people who crave the behaviour associated with consumption of combustible tobacco - the so-called hand-to-mouth aspect - of smoking tobacco. Inhalators generally allow nicotine-containing aerosol to be inhaled through an elongate tube in which a container containing a nicotine carrier, for example, a substrate, is located. An air stream caused by suction through the tube by the user carries nicotine vapours into the lungs of the user to satisfy a nicotine craving. The container may comprise a replaceable cartridge, which includes a cartridge housing and a passageway in the housing in which a nicotine reservoir is located. The reservoir holds a measured amount of nicotine in the form of the nicotine carrier. The measured amount of nicotine is an amount suitable for delivering a specific number of "doses". The form of the nicotine carrier is such as to allow nicotine vapour to be released into a fluid stream passing around or through the reservoir. This process is known as aerosolization and or atomization. Aerosolization is the process or act of converting a physical substance into the form of particles small and light enough to be carried on the air i.e. into an aerosol. Atomization is the process or act of separating or reducing a physical substance into fine particles and may include the generation of aerosols. The passageway generally has an opening at each end for communication with the exterior of the housing and for allowing the fluid stream through the passageway. A nicotine-impermeable barrier seals the reservoir from atmosphere. The barrier includes passageway barrier portions for sealing the passageway on both sides of the reservoir. These barrier portions are frangible so as to be penetrable for opening the passageway to atmosphere.

A device or a system that is powered can fall into two sub-categories. In both subcategories, such devices or systems may comprise electronic devices or systems that permit a user to simulate the act of smoking by producing an aerosol mist or vapour that is drawn into the lungs through the mouth and then exhaled. The electronic devices or systems typically cause the vaporization of a liquid containing nicotine and entrainment of the vapour into an airstream. Vaporization of an element or compound is a phase transition from the liquid phase to vapour i.e. evaporation or boiling. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

A person of ordinary skill in the art will appreciate that devices or systems of the second, powered category as used herein include, but are not limited to, electronic nicotine delivery systems, electronic cigarettes, e-cigarettes, e-cigs, vaping cigarettes, pipes, cigars, cigarillos, vaporizers and devices of a similar nature that function to produce an aerosol mist or vapour that is inhaled by a user. Such nicotine delivery devices or systems of the second category incorporate a liquid reservoir element generally including a vaporizer or misting element such as a heating element or other suitable element, and are known, inter alia, as atomizers, cartomizers, or clearomizers. Some electronic cigarettes are disposable; others are reusable, with replaceable and refillable parts.

Aerosol delivery devices or systems in a first sub-category of the second, powered category generally use heat and/or ultrasonic agitation to vaporize a solution comprising nicotine and/or other flavouring, propylene glycol and/or glycerine-based base into an aerosol mist of vapour for inhalation.

Aerosol delivery devices or systems in a second sub-category of the second, powered category may typically comprise devices or systems in which tobacco is heated rather than combusted. During use, volatile compounds may be released from the tobacco by heat transfer from the heat source and entrained in air drawn through the aerosol delivery device or system. Direct contact between a heat source of the aerosol delivery device or system and the tobacco heats the tobacco to form an aerosol. As the aerosol containing the released compounds passes through the device, it cools and condenses to form an aerosol for inhalation by the user. In such devices or systems, heating, as opposed to burning, the tobacco may reduce the odour that can arise through combustion and pyrolytic degradation of tobacco.

Aerosol delivery devices or systems falling into the first sub-category of powered devices or systems may typically comprise a powered unit, comprising a heater element, which is arranged to heat a portion of a carrier that holds an aerosol precursor. The carrier comprises a substrate formed of a "wicking" material, which can absorb aerosol precursor liquid from a reservoir and hold the aerosol precursor liquid. Upon activation of the heater element, aerosol precursor liquid in the portion of the carrier in the vicinity of the heater element is vaporised and released from the carrier into an airstream flowing around the heater and carrier. Released aerosol precursor is entrained into the airstream to be borne by the airstream to an outlet of the device or system, from where it can be inhaled by a user.

The heater element is typically a resistive coil heater, which is wrapped around a portion of the carrier and is usually located in the liquid reservoir of the device or system. Consequently, the surface of the heater may always be in contact with the aerosol precursor liquid, and long-term exposure may result in the degradation of either or both of the liquid and heater. Furthermore, residues may build up upon the surface of the heater element, which may result in undesirable toxicants being inhaled by the user. Furthermore, as the level of liquid in the reservoir diminishes through use, regions of the heater element may become exposed and overheat.

The present invention has been devised in light of the above considerations.

### Summary of the Invention

At its most general, the present invention proposes that an aerosol generation apparatus has a mesh which receives aerosol precursor from a reservoir. The user is able to draw air through a duct to the mesh, the passage of air through the mesh causing the aerosol precursor to pass from the mesh in atomised form. The atomised aerosol precursor from the mesh sprays onto the heater to be vaporised, and flow through the duct to the user. Hence, the user controls the atomisation and vapourisation of the aerosol precursor as they draw on the apparatus.

The duct will normally extend from an air inlet of the apparatus to a mouthpiece of the apparatus, so that the user draws air from the inlet to the mouthpiece via the mesh.

There is also preferably a wick between the reservoir and the mesh, for regulating the transfer of aerosol precursor from the reservoir to the mesh.

Thus, the present invention may provide an aerosol-generation apparatus comprising;
a reservoir for holding an aerosol precursor; an air duct passing from an air inlet of said apparatus to a mouthpiece of said apparatus, thereby to allow a user to draw air through said air duct from said inlet to said mouthpiece; a mesh through which said air duct passes, said mesh being arranged to receive said aerosol precursor from said reservoir, and atomise said aerosol precursor on said mesh due to passage of air through said mesh, thereby to form a spray of atomised aerosol precursor; a heater arranged to receive said spray of atomized aerosol precursor from said mesh and to heat and thereby vaporise said atomized aerosol precursor to form a mixture of vapour and air in said air duct at said heater; wherein said mouthpiece is arranged to receive said mixture from said heater via said air duct.

Note that it is not necessary that all of the aerosol precursor reaching the mesh is atomised, and not necessary that all the atomised aerosol precursor is vapourised by the heater. The mixture may itself be mixed with some un-atomised aerosol precursor and/or some atomised but un-vapourised aerosol precursor as it passes to the mouthpiece. However, it is desirable for the atomisation/vaporisation processes to be efficient, to maximise the amount of vapour passing to the user, although those processes need not be complete.

Preferably, the apparatus further includes a wick between said reservoir and said mesh, said wick being arranged to receive aerosol precursor from said reservoir and to transfer said aerosol precursor to said mesh. The wick may be of a fibrous material, such as glass fibre, cotton or ceramic fibre, may be of porous polymeric material, or may be of porous ceramic material.

The reservoir may be a simple tank to hold liquid aerosol precursor, but optionally at least a part of the reservoir is a porous polymer material, which porous polymer material transfers aerosol precursor to the wick.

In another aspect of the present invention, there may be provided an aerosol delivery system in which the aerosol-generation apparatus described above is mounted in two separable housings. The first housing has therein the reservoir, the mouthpiece, the mesh and parts of the air duct. The second housing has therein the heater and other parts of the air duct. Thus, the reservoir may be separated from the heater by separating the housings, to enable the reservoir to be refilled or replaced.

The wick, if present, will normally be in the first housing.

Preferably, the air inlet is in the second housing, the air inlet leading to the parts of the duct in the second housing, to enable air to enter the duct from the exterior of the aerosol delivery system. The duct then extends from said inlet to said first housing.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
**Figure 1** is a perspective view illustration of a system for aerosol delivery according to one or more embodiments of the present invention;
**Figure 2** is a cross-section side view illustration of part of an apparatus of the system for aerosol delivery of Figure 1;
**Figure 3** is a cross-section side view illustration of the system and apparatus for aerosol delivery of Figure 1;
**Figure 4** is a perspective view illustration of an aerosol carrier for use in the system for aerosol delivery according to one or more embodiments of the present invention;
**Figure 5** is a cross-section side view of part of the system and apparatus of Figures 1 to 4;
**Figure 6** is an exploded perspective view illustration of a kit-of-parts for assembling the system according to one or more embodiments of the present invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

In general outline, one or more embodiments in accordance with the present invention may provide a system for aerosol delivery in which an aerosol carrier may be inserted into a receptacle (e.g. a "heating chamber") of an apparatus for initiating and maintaining release of an aerosol from the aerosol carrier. Another end, or another end portion, of the aerosol carrier may protrude from the apparatus and can be inserted into the mouth of a user for the inhalation of aerosol released from the aerosol carrier cartridge during operation of the apparatus.

Hereinafter, and for convenience only, "system for aerosol delivery" shall be referred to as "aerosol delivery system".

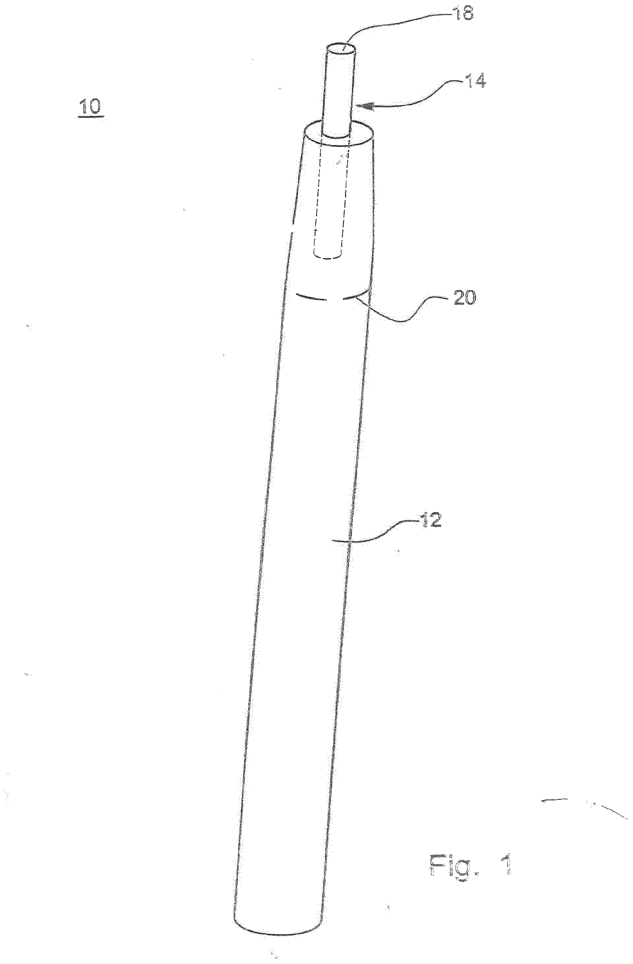

Referring now to Figure 1, there is illustrated a perspective view of an aerosol delivery system 10 comprising an aerosol generation apparatus 12 operative to initiate and maintain release of aerosol from a fluid-transfer article in an aerosol carrier 14. In the arrangement of Figure 1, the aerosol carrier 14 is shown with a first end thereof and a portion of the length of the aerosol carrier 14 located within a receptacle of the apparatus 12. A remaining portion of the aerosol carrier 14 extends out of the receptacle. This remaining portion of the aerosol carrier 14, terminating at a second end 18 of the aerosol carrier, is configured for insertion into a user's mouth. The second end 18 thus acts as a mouthpiece. A vapour is produced when a heater (not shown in Figure 1) of the apparatus 12 heats the aerosol carrier 14 to form a mixture of vapour and air, and this can be atomised aerosol precursor, and this can be delivered to the user, when the user sucks or inhales, via a fluid passage (air duct) in communication with an outlet of the aerosol carrier 14 from the fluid-transfer article to the second end 18.

The device 12 also comprises air-intake apertures 20 in the housing of the apparatus 12 acting as inlets to provide a passage for air to be drawn into the interior of the apparatus 12 (when the user sucks or inhales) for delivery to the first end 16 of the aerosol carrier 14, so that the air can be drawn into the device. Optionally, these apertures may be perforations in the housing of the apparatus 12.

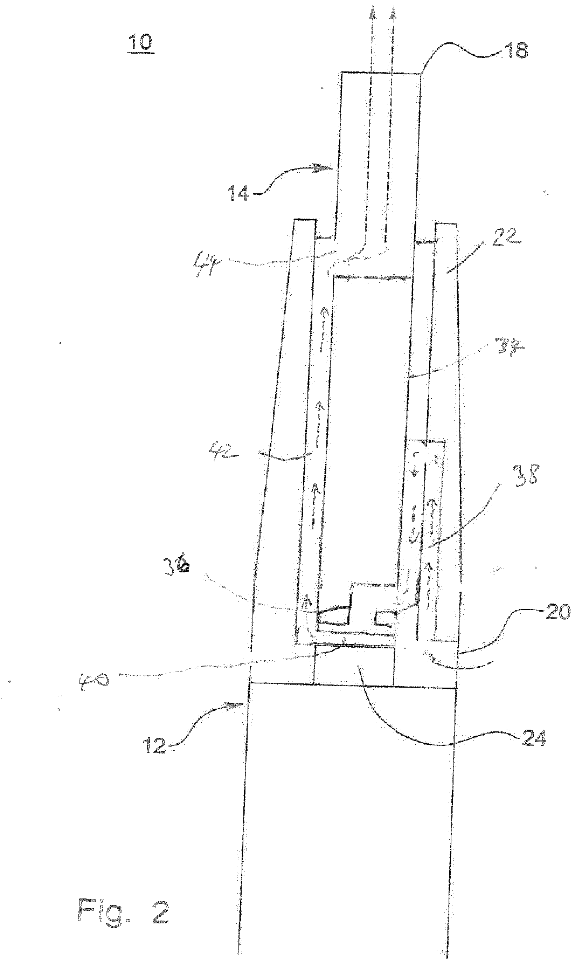

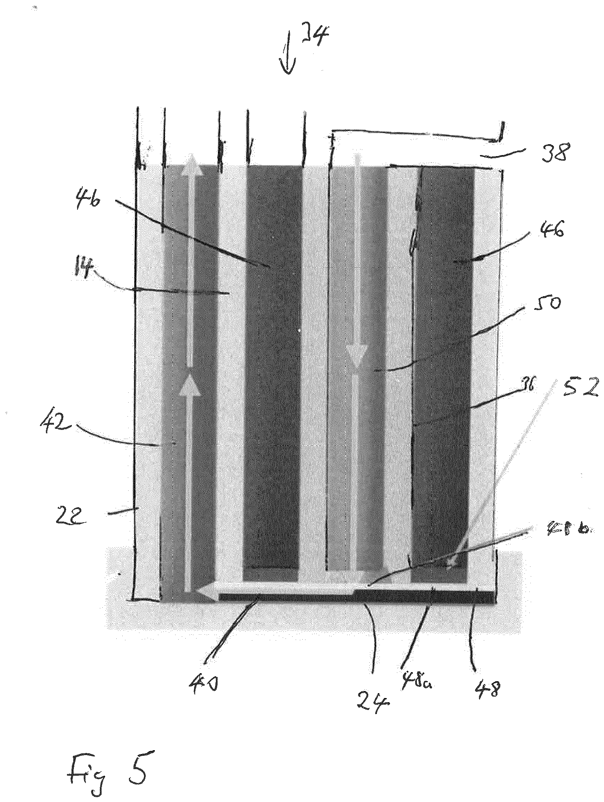

A fluid-transfer article 34 (not shown in Figure 1, but described hereinafter with reference to Figures 2 and 5 is located within a housing of the aerosol carrier 14. The fluid-transfer article contains an aerosol precursor material, which may include at least one of: nicotine; a nicotine precursor material; a nicotine compound; and one or more flavourings. The fluid-transfer article is located within the housing of the aerosol carrier 14.

The substrate of the fluid-transfer article 34 may comprise a porous material where pores of the porous material hold, contain, carry, or bear the aerosol precursor material and thus act as a reservoir for the aerosol precursor. In particular, the porous material of the fluid-transfer article may be a porous polymer material such as, for example, a sintered material. Particular examples of material suitable for the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex®. Further optionally, a substrate forming the fluid-transfer article may comprise Polypropylene (PP) or Polyethylene Terephthalate (PET). All such materials may be described as heat resistant polymeric wicking material in the context of the present invention.

Alternatively, the fluid-transfer article 34 may comprise a simple liquid reservoir in the form of an empty tank for the receipt and storage of liquid aerosol precursor, rather than a porous material for holding the aerosol precursor.

The aerosol carrier 14 is removable from the apparatus 12 so that it may be disposed of when expired. After removal of a used aerosol carrier 14, a replacement aerosol carrier 14 can be inserted into the apparatus 12 to replace the used aerosol carrier 14.

Figure 2 is a cross-sectional side view illustration of a part of apparatus 12 of the aerosol delivery system 10. The apparatus 12 comprises a receptacle 22 in which is located a portion of the aerosol carrier 14. In one or more optional arrangements, the receptacle 22 may enclose the aerosol carrier 14. The aerosol carrier 14 and the receptacle 22 are separable, so they may be considered first and second housings. The apparatus 12 also comprises a heater 24, which opposes an atomiser 36 of the fluid-transfer article 34 when the aerosol carrier 14 is located within the receptacle 22.

Air flows into the apparatus 12 (in particular, into a closed end of the receptacle 22) via air-intake apertures 20. From the closed end of the receptacle 22, the air is drawn through a duct 38 into the atomiser 36. The internal components of the atomiser 36, and the fluid therethrough, will be described later with reference to Figure 5. The air passes through the atomiser 36 and out into a gap 40 between the atomiser 36 and the heater 24. This will be described in more detail later, but it can be noted that the gap 40 acts as an activation region for aerosol precursor. From gap 40 the air passes through a further duct 42 in the receptacle 22, through an opening 34 in the aerosol carrier 14, and may then pass to the second end 18, which acts as a mouthpiece. The air flow is as illustrated by the dotted arrows in Figure 2. In effect, a continuous air duct is formed from the air-intake apertures 20 to the second end 19, through the duct 38, through the atomiser 36, through the gap 40, through the further duct 42, through the opening 44 and through the carrier 14 to the mouthpiece. When the user draws air from the mouthpiece, air flow is generated along the whole of that air duct.

To achieve release of the captive aerosol from the fluid-transfer article, aerosol precursor is released from a reservoir (not shown in Figure 2) of the fluid transfer article 34, to the atomiser 36 and passes to the heater 24 where it is heated. As a user sucks or inhales on the second end 18 of the aerosol carrier 14, the aerosol released from the fluid-transfer article 34 is entrained in the air flowing through the atomiser 36 to be atomised and subsequently vapourised by the heater 24, and is then drawn through the ducts 42 towards the second end 18 and onwards into the user's mouth.

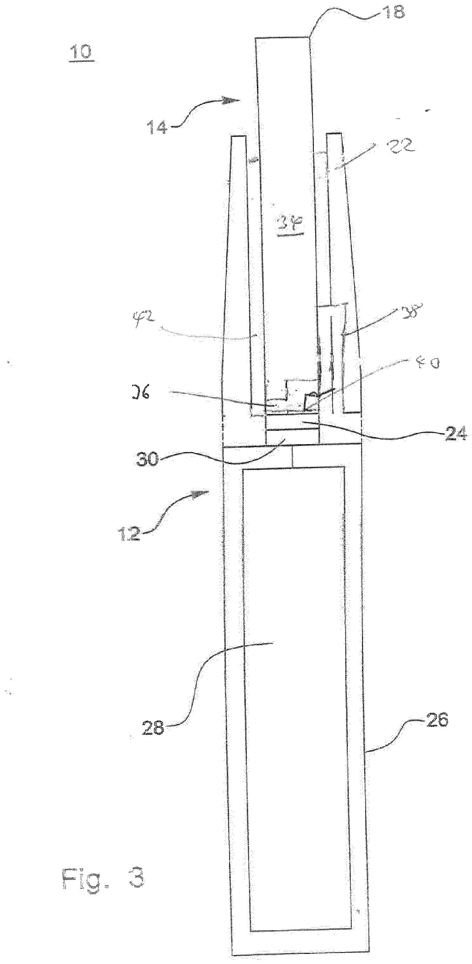

Turning now to Figure 3, a cross-sectional side view of the aerosol delivery system 10 is schematically illustrated showing the features described above in relation to Figures 1 and 2 in more detail. As can be seen, apparatus 12 comprises a housing 26, which may support or be integral with the receptacle 22. The housing 26 also contains control circuitry (not shown) operative by a user, or upon detection of air and/or vapour being drawn into the device 12 through air-intake apertures 20, i.e. when the user sucks or inhales. Additionally, the housing 26 comprises an electrical energy supply 28, for example a battery. Optionally, the battery comprises a rechargeable lithium ion battery. The housing 26 also comprises a coupling 30 for electrically (and optionally mechanically) coupling the electrical energy supply 28 to control circuitry (not shown) for powering and controlling operation of the heater 24.

Responsive to activation of the control circuitry of the apparatus 12, the heater 24 heats the activation region in gap 40. This heating process initiates (and, through continued operation, maintains) vaporisation of atomised aerosol precursor on the heater 24 and/or present in the gap 40. The vapour formed as a result of the heating process is entrained into a stream of air being drawn through the activation region formed by the gap 40 (as the user sucks or inhales). The stream of air with the entrained vapour passes through the duct 42 and exits the aerosol carrier 14 at second end 18 for delivery to the user. This process is briefly described above in relation to Figure 2, where arrows schematically denote the flow of the air stream into the device 12 and through the aerosol carrier 14, and the flow of the air stream with the entrained vapour and/or aerosol through the aerosol carrier 14.

Note that, as previously mentioned, the atomisation of aerosol precursor by the atomiser 36 and/or the vapourisation of atomised aerosol precursor by the heater 24 need not be complete. Therefore, there may be un-atomised or un-vapourised aerosol precursor in the air flow at gap 40 and/or in the duct 42, which may then pass to the second end 18. The mixture of air and vapour which passes to the user may itself be mixed with some un-atomised or un-vapourised aerosol precursor, although some of that aerosol precursor may stick to the sides of the duct 42 and flow back towards the heater 24. In general, it is desirable that the amount of un-atomised and/or un-vapourised aerosol precursor is small.

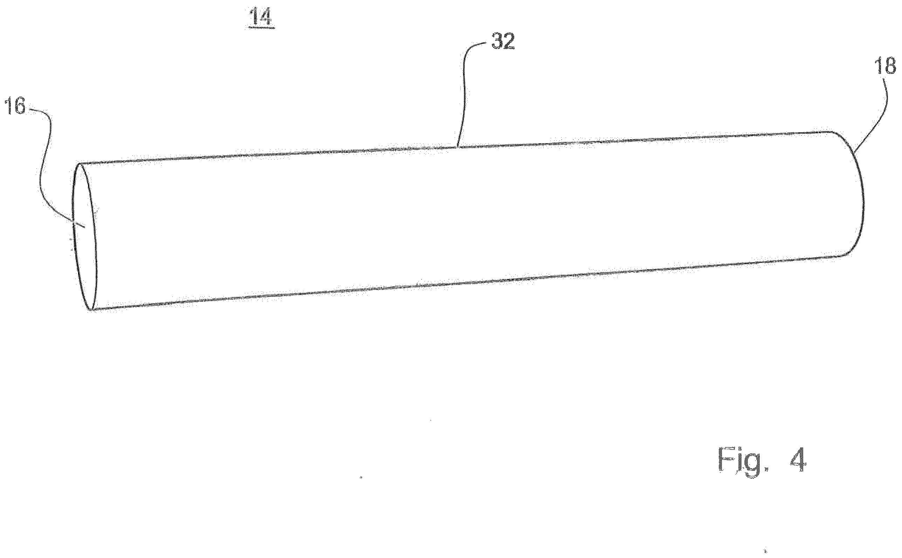

Figures 4 and 5 schematically illustrate the aerosol carrier 14 in more detail, and Figure 5 illustrates features within the receptacle in more detail. Figure 4 illustrates an exterior of the aerosol carrier 14, and Figure 5 illustrates internal components in one optional configuration.

Fig. 4 illustrates the exterior of the aerosol carrier 14, which comprises housing 32 for housing said fluid-transfer article (not shown). The particular housing 32 illustrated in Figure 4 comprises a tubular member, which may be generally cylindrical in form, and which is configured to be received within the receptacle of the apparatus. Of course, in other arrangements the housing 32 need not be cylindrical and can take alternative forms. The first end 16 of the aerosol carrier 14 is for location to oppose the intermediate structure and the heater 24 of the apparatus, and the second end 18 (and the region adjacent the second end 18) is configured for insertion into a user's mouth.

Figure 5 illustrates some internal components of the aerosol carrier 14, atomiser 36 and the heater 24 of apparatus 12, in in one proposed embodiment of the invention.

As described above, the aerosol carrier 14 comprises a fluid-transfer article 34. The fluid-transfer article 34 may be removable from the carrier 14, to enable it to be replaced. The fluid-transfer article 34 acts as a reservoir for aerosol precursor and that aerosol precursor will be consumed as the apparatus is used. Once sufficient aerosol precursor has been consumed, the aerosol precursor will need to be replaced. It may then be easiest to replace it by replacing the fluid-transfer article 34, rather than trying to re-fill the fluid-transfer article 34 with aerosol precursor while it is in the carrier 14.

Figure 5 then illustrates in more detail parts of the fluid-transfer article 34, which includes the atomiser 36, the heater 24 and adjacent ducts and housings. The fluid-transfer article has a reservoir 46 which may, as previously described, be of porous material holding the aerosol precursor, or may be a tank for the storage of liquid aerosol precursor. The atomiser 36 then comprises a duct 50, a mesh 48, and a wick 52. In this arrangement, the atomiser 36 is part of the fluid-transfer article 34, and contained within the carrier 14. The reservoir 46 extends around the duct 50.

The wick 52 is in contact with the reservoir 46 to receive aerosol precursor therefrom. It is also in contact with an outer part 48a of the mesh 48, so that aerosol precursor is transferred via the wick 52 to that outer part 48a of the mesh 48. Further wicking action within the mesh 48 itself causes the aerosol precursor to transfer from the outer part 48a of the mesh 48 to an inner part 48b of the mesh 48, that inner part 48b being aligned with the duct 50. The air path from the duct 50 passes through the mesh 48, and flow of air through the duct 50 causes liquid at the inner part 46b of the mesh to be expelled from the mesh 48 in atomised form. In particular, as the air flows through the mesh 48, it forces the aerosol precursor from the voids in the mesh 48, which generates atomised aerosol precursor in the air flow. The atomised aerosol precursor crosses the gap 40 onto the heater 24 to be vaporised by the heat therefrom.

Whilst it would be possible for the mesh 48 to receive aerosol precursor directly from the reservoir 46, the presence of the wick 52 is advantageous, as it controls or regulates the flow of liquid onto the mesh, so that the mesh does not become over-saturated. The amount of aerosol precursor reaching the mesh will be determined by the degree of wicking occurring within the wick 52. Thus, the amount of aerosol precursor reaching the central region 48b of the mesh 48 may be regulated by the wick 52 so that substantially all of the aerosol precursor at that central region 48b will be atomised by the air flow from the duct 50. Similarly, the amount of aerosol precursor which has been atomised at the mesh 48 can be regulated (for example, by choice of the sizes of the duct 50 and the central region 48 of the mesh 48, so that substantially all of the atomised aerosol precursor is vaporised by the heater 24. It is not necessary that the atomisation and/or vaporisation processes are complete, although it is preferable that they are as efficient as possible.

As aerosol precursor is removed from the central region 46b of the mesh 48 further aerosol precursor will transfer from the outer region 46a, and similarly additional aerosol precursor will then pass to the outer region 48a and the wick 52. Such removal of the aerosol precursor from the wick 52 causes more aerosol precursor to be drawn from the reservoir 46, to maintain the wick 52 in a saturated or semi-saturated state.

In the arrangement shown in Figure 5, the mesh 48 and the wick 52 are at an end of the casing 14, and thus part of with the fluid-transfer article 34. The duct 50 also forms part of the structure. Thus, when the carrier 14 is removed from the receptacle 22, the mesh 48 and wick 52 are also removed, but the heater 24 remains. As an alternative, it may be possible for the mesh 48 to be supported by the receptacle 22, and be separable from the casing 14. In such an arrangement, it will normally be preferable for the wick 52 to be removed with the casing 14, as the wick 52 closes the reservoir 46.

Thus, with the present invention, the passage of air through the central region 48b of the mesh 48 from the duct 50 generates a spray of atomised aerosol precursor in the gap 40, with the movement of air causing that spray to impinge on the heater 24. The heater 24 then heats the atomised aerosol precursor deposited thereon, to vaporise at least some of the atomised aerosol precursor at the activation region formed by the gap 40. A mixture of vapour and air, or a mixture of vapour, aerosol, and air then passes through the duct 42, and ultimately to the second end 18 forming the mouthpiece, as was described with reference to Figure 2. In practice, a plurality of ducts 38 and ducts 42 may be provided at different radial positions around the fluid-transfer article 34, via multiple air flow paths to the venturi 36 and to the second end 18.

In the arrangement of Figure 5, there is no direct contact between the mesh 48 and the heater 24. Instead, the atomised aerosol precursor is heated by being sprayed from the mesh 48 on to the heater. This means that the mesh 48 can be made of a material with less heat resistance then would be needed if it was in contact with the heater 24. Similarly the wick 52 can be made of less expensive material. For example, the wick 52 may be of a fibrous material, such as glass fibre, cotton or ceramic fibre. Alternatively, it may be a porous polymer material, such as a sintered polymer, or a porous ceramic material, such as a sintered ceramic. The separability of the carrier 14 from the rest of the device enables the aerosol precursor in the reservoir 46 to be replaced as it is consumed during use of the device. This may be done by re-filling the reservoir 46 or replacing the fluid-transfer article 34 with another such article having a filled reservoir 46. The heater 24 thus remains with the device even when the reservoir 46 is removed and the fluid-transfer article replaced, optimising the cost of the fluid-transfer article 34.

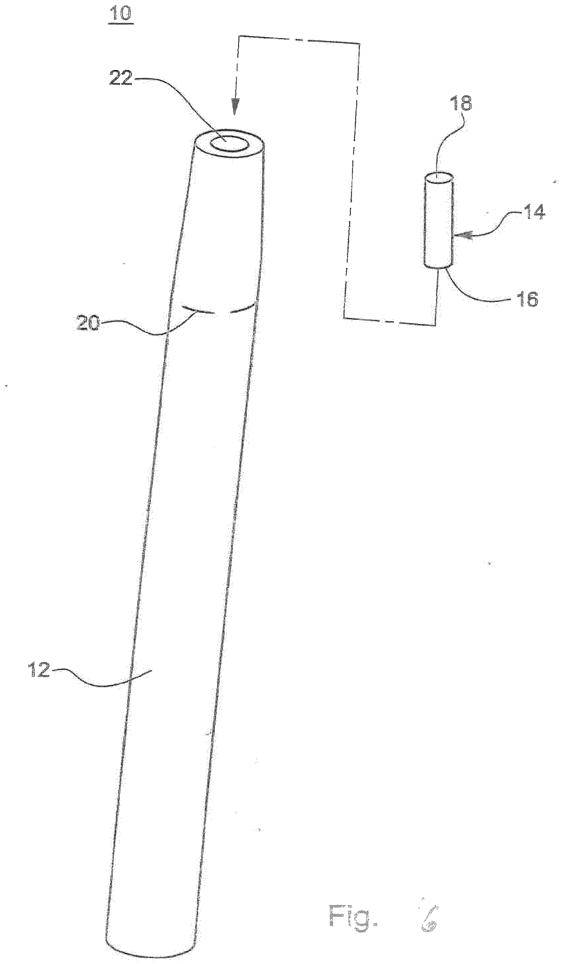

Figure 6 is an exploded perspective view illustration of a kit-of-parts for assembling an aerosol delivery system 10.

As will be appreciated, in the arrangements described above, the fluid-transfer article 34 is provided within a housing 32 of the aerosol carrier 14. In such arrangements, the housing of the carrier 14 serves to protect the aerosol precursor-containing fluid-transfer article 34, whilst also allowing the carrier 14 to be handled by a user without his/her fingers coming into contact with the aerosol precursor liquid retained therein.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An aerosol-generation apparatus comprising;
a reservoir for holding an aerosol precursor;
an air duct passing from an air inlet of said apparatus to a mouthpiece of said apparatus, thereby to allow a user to draw air through said air duct from said inlet to said mouthpiece; a mesh through which said air duct passes, said mesh being arranged to receive said aerosol precursor from said reservoir, and atomise said aerosol precursor on said mesh due to passage of air through said mesh, thereby to form a spray of atomised aerosol precursor from said mesh.
a heater arranged to receive said spray of atomized aerosol precursor from said mesh and to heat and thereby vaporise said atomized aerosol precursor to form a mixture of vapour and air in said air duct at said heater;
wherein said mouthpiece is arranged to receive said mixture from said heater via said air duct.

2. An aerosol-generation apparatus according to claim 1, further including a wick between said reservoir and said mesh, said wick being arranged to receive aerosol precursor from said reservoir and to transfer said aerosol precursor to said mesh.

3. An aerosol-generation apparatus according to claim 2, wherein said wick is of fibrous material.

4. An aerosol-generation apparatus according to claim 2, wherein said wick is of porous polymeric material.

5. An aerosol-generation apparatus according to claim 2, wherein said wick is of porous ceramic material.

6. An aerosol-generation apparatus according to any one of the preceding claims, wherein at least a part of said reservoir is of porous polymer material.

7. An aerosol delivery system having an aerosol-generation apparatus according to any one of the preceding claims, a first housing having therein said reservoir, said mouthpiece, said mesh and parts of said air duct, and a second housing having therein said heater, and other parts of said air duct, said first and second housings being separable.

8. An aerosol delivery system according to claim 7 as dependent on claim 2, wherein said first housing also has said wick therein.

9. An aerosol delivery system according to any one of the claims 7 or 8, wherein said air inlet is in said second housing.
